# EUROPEAN PATENT SPECIFICATION

(11) **EP 4 334 023 B1**
(45) Date of publication and mention of the grant of the patent: **02.04.2025**
(21) Application number: 22725627.8
(22) Date of filing: 05.05.2022
(51) Int. Cl.: B01F 21/00, B01F 27/90, B01F 35/90, B01F 21/10

(54) **METHODS OF IN VITRO DISSOLUTION**
VERFAHREN ZUR IN-VITRO-AUFLÖSUNG
PROCÉDÉS DE DISSOLUTION IN VITRO

(30) Priority: 05.05.2021 US 202163184629 P
(43) Date of publication of application: 13.03.2024
(73) Proprietor: Bristol-Myers Squibb Company, Princeton, NJ 08543 (US)
(72) Inventor: LU, Xujin, Princeton, New Jersey 08543 (US); LO, Lili, Princeton, New Jersey 08543 (US)
(74) Representative: Mewburn Ellis LLP
(86) International application number: PCT/US2022/027910
(87) International publication number: WO 2022/235963

(56) References cited:
- KR-B1- 102 219 467
- US-A1- 2006 260 423

## Description

### FIELD OF THE DISCLOSURE

The present disclosure provides methods of *in vitro* dissolution of a solute, *e.g.*, a long acting injectable.

### BACKGROUND OF THE DISCLOSURE

Long acting injectables (LAIs) are formulations that show an extended release profile over days or weeks. They present an important and often a realistic approach for exploiting the therapeutic value of many development candidates to reduce dosing frequency and improve patient compliance. During development of LAI formulations, as well as assessment of the drug product batch-to-batch quality, *in vitro* drug release and dissolution testing play important roles with increasing interests and expectations from both regulatory agencies and industry.

Currently, the most used methods for LAI in-vitro dissolution are (1) sample and separate methods, (2) dialysis methods, and (3) flow-through methods. These methods have been applied generally well to small molecule LAIs, but these methods have had limited success when applied to large molecule such as peptides. As such, there remains a need in the art for dissolution methods that exhibit ruggedness and discriminating ability with respect to critical quality attributes, and that are capable of predicting *in vivo* performance. US 2006/260423 A1 discloses an apparatus and method comprising agitating a solution in a vessel using a submerged mobile paddle wherein the solution comprises a solute and a media. KR 102 219 467 B1 discloses a dissolution apparatus and method wherein a permeable barrier supports the solute.

### SUMMARY OF THE DISCLOSURE

Some aspects of the present disclosure are directed to an *in vitro* dissolution method comprising agitating a solution placed in a vessel using a mobile paddle; wherein the solution comprises a solute, a media, and a plurality of beads; wherein the plurality of beads is positioned between the solute and the mobile paddle; and wherein the mobile paddle is submerged in the solution.

In some aspects, the method comprises, prior to the agitating, loading the solution into the vessel by: (i) adding the solute to the bottom of the vessel; (ii) adding the plurality of beads to the bottom of the vessel, wherein the plurality of beads are added on top of the solute; and adding a volume of media to the vessel, wherein the media is added on top of the solute and the plurality of beads.

In some aspects, the media is added without agitating the solute or the plurality of beads. In some aspects, the mobile paddle is not in contact with the plurality of beads or the solute while the mobile paddle is in a stationary position.

In some aspects, the solute comprises a long acting injectable. In some aspects, the solute comprises a long acting injectable microsphere.

In some aspects, the plurality of beads comprises one or more glass beads, one or more plastic beads, one or more silicate beads, one or more metal beads, or any combination thereof. In some aspects, the plurality of beads comprises one or more beads having a diameter of at least about 0.01 mm, at least about 0.05 mm, at least about 0.1 mm, at least about 0.5 mm, at least about 1.0 mm, at least about 1.5 mm, or at least about 2.0 mm. In some aspects, the plurality of beads comprises one or more beads having a diameter of about 1.0 mm.

In some aspects, the volume of the solution in the vessel is sufficient to submerge the mobile paddle, the plurality of beads, and the solute. In some aspects, the volume of the solution in the vessel is at least about 50 mL, at least about 60 mL, at least about 70 mL, at least about 75 mL, at least about 80 mL, at least about 90 mL, or at least about 100 mL.

In some aspects, the plurality of beads comprises at least about 3 g of beads per about 100 mL of solution, at least about 4 g of beads per 100 mL of about solution, at least about 5 g of beads per about 100 mL of solution, at least about 6 g of beads per about 100 mL of solution, or at least about 7 g of beads per about 100 mL of solution. In some aspects, the plurality of beads comprises about 4 g of beads per 100 mL of solution. In some aspects, the plurality of beads comprises less than about 6 g of beads per 100 mL of solution.

In some aspects, the solute comprises a long acting injectable microsphere loaded with a biologically active moiety.

In some aspects, the method further comprises applying a cover the vessel during dissolution. In some aspects, the cover reduces or eliminates loss of evaporated solution during dissolution.

In some aspects, the pH of the solution is adjusted.

In some aspects, an additional volume of the plurality of beads is added below the solute.

In some aspects, the method comprises: (i) adding a solute to the bottom of a vessel, wherein the solute comprises a long acting injectable microsphere; (ii) adding a plurality of beads on top of the solute, wherein the plurality of beads comprises one or more glass beads; (iii) adding a media to the solute and the volume of beads, wherein the media is added without agitating the solute or the volume of beads, and wherein the media is added at a volume that is sufficient to submerge the mobile paddle; and (iv) applying a force to cause the mobile paddle to spin and/or oscillate, agitating the solution.

In some aspects, the method comprises: (i) adding a first plurality of beads to the bottom of a vessel, wherein the plurality of beads comprises one or more glass beads; (ii) adding a solute on top of the plurality of beads, wherein the solute comprises a long acting injectable microsphere; (iii) adding a second plurality of beads on top of the solute, wherein the plurality of beads comprises one or more glass beads; (iv) adding a media to the solute and the volume of beads, wherein the media is added without agitating the solute or the volume of beads, and wherein the media is added at a volume that is sufficient to submerge the mobile paddle; and (v) applying a force to cause the mobile paddle to spin and/or oscillate, agitating the solution.

In some aspects, the method further comprises adding a volume of a vehicle solution to the solute and/or the plurality of beads, wherein the volume of the vehicle solution is sufficient to wet but not completely submerge the solute and/or the plurality of beads.

In some aspects, the method further comprises detecting the presence of dissolved solute in the media. In some aspects, the detecting comprises inserting a probe into the solution, obtaining a sample of the solution, or a combination thereof. In some aspects, the presence of dissolved solute in the media is detected by probing and/or obtaining a sample from a portion of the solution that is above the mobile paddle.

In some aspects, the solute comprises a long acting injectable comprising a biologically active moiety. In some aspects, the biologically active moiety comprises a small molecule or a polypeptide. In some aspects, the polypeptide has a molecular weight of less than about 10 kDa, less than about 9 kDa, less than about 8 kDa, less than about 7 kDa, less than about 6 kDa, less than about 5 kDa, or less than about 4 kDa. In some aspects, the polypeptide has a molecular weight of about 4 kDa.

In some aspects, the solution further comprises a surfactant. In some aspects, the surfactant is selected from the group consisting of polysorbate 20, polysorbate 80, TRITON X100, PLURONIC F-68, and any combination thereof. In some aspects, the solution further comprises an antimicrobial agent.

Some aspects of the present disclosure are directed to a kit comprising: a vessel; a plurality of beads; a media; a mobile paddle, wherein the mobile paddle can be positioned inside the vessel; an optional cover; and instructions for dissolving a solute according any method disclosed herein.

### BRIEF DESCRIPTION OF THE DRAWINGS

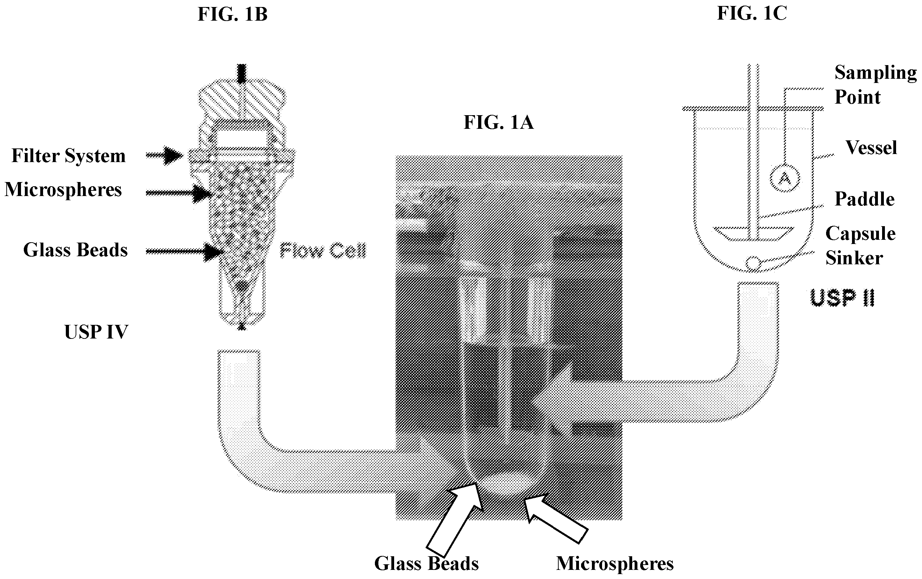

**FIG. 1A** is a schematic representation of the *in vitro* dissolution method disclosed herein, wherein an LAI sample sandwiched in glass beads at the bottom of the vessel, and a mobile paddle is positioned over the glass beads. Previous methods are exemplified in FIG. 1B ("USP IV") and FIG. 1C ("USP II").

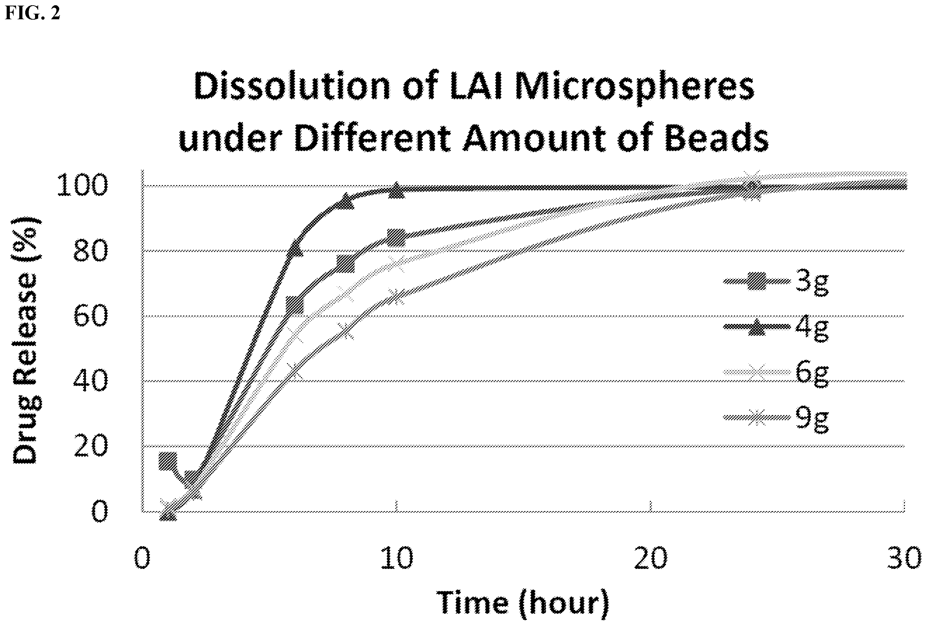

**FIG. 2** is a graphical representation of dissolution of drug product from LAI (y-axis) over time (x-axis), wherein the LAI is positioned under 3 g (squares), 4 g (triangles), 6 g (x's) or 9 g (stars) of glass beads (1 mm diameter).

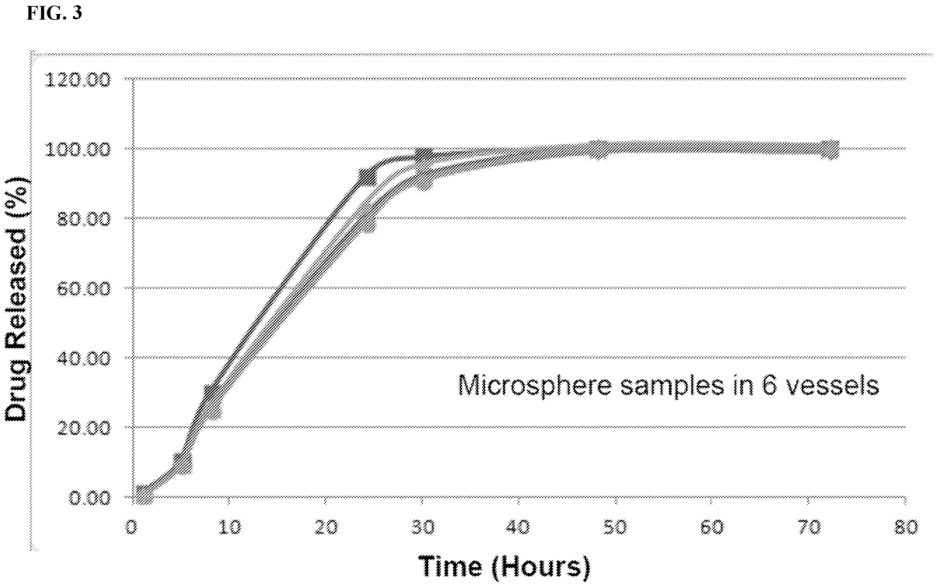

**FIG. 3** is a graphical representation of dissolution of drug product from LAI (y-axis) over time (x-axis), wherein the LAI is positioned under 4 g of glass beads (1 mm diameter) for 6 runs, in 6 vessels.

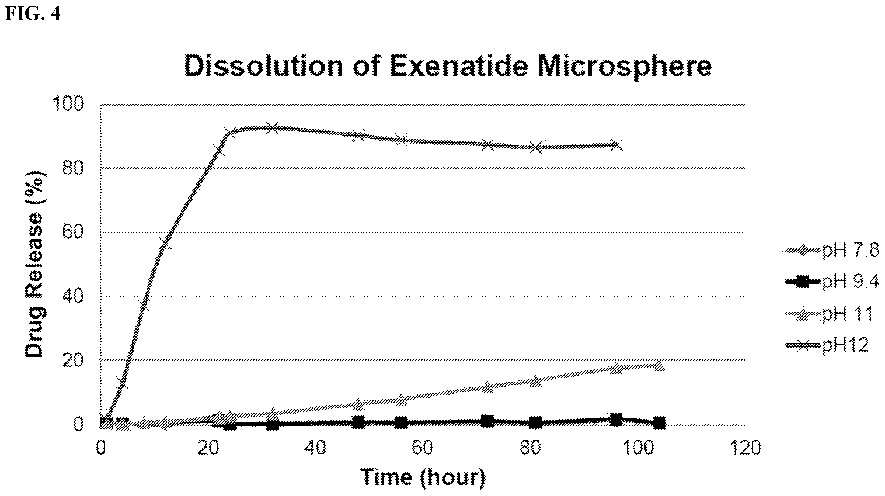

**FIG. 4** is a graphical representation of dissolution of drug product, exenatide, from LAI microspheres (y-axis) over time (x-axis), wherein the LAI is positioned under 4 g of glass beads (1 mm diameter) at 37 °C, wherein the pH is held at pH 7.8 (diamonds), pH 9.4 (squares), pH 11 (triangles), or pH 12 (x's).

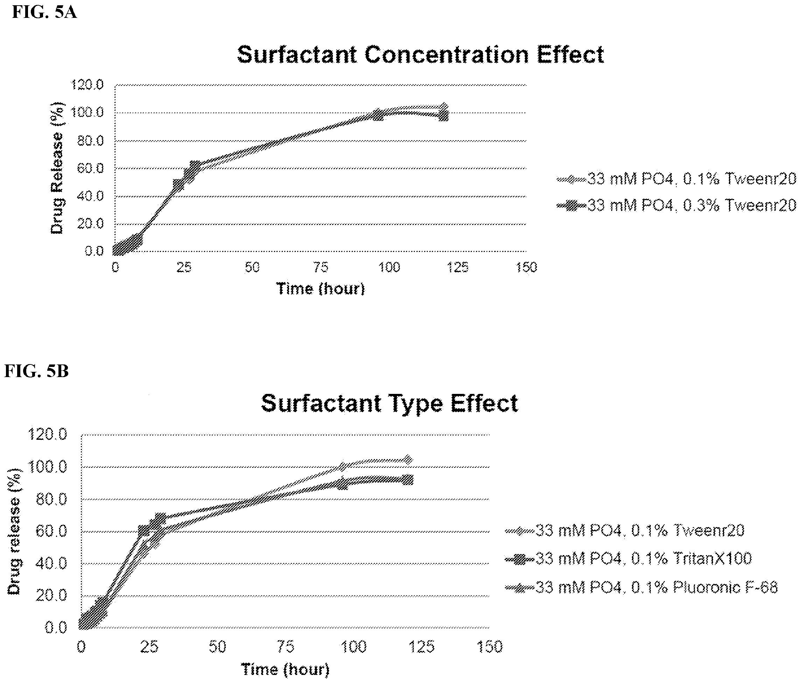

**FIG. 5A** is a graphical representation of dissolution of drug product, exenatide, from LAI microspheres (y-axis) over time (x-axis), wherein the LAI is positioned under 4 g of glass beads (1 mm diameter) at 37 °C, wherein the buffer comprises 33 mM PO₄ and 0.1% TWEEN20 (diamonds) or 0.3% TWEEN20 (squares). **FIG. 5B** is a graphical representation of dissolution of drug product, exenatide, from LAI microspheres (y-axis) over time (x-axis), wherein the LAI is positioned under 4 g of glass beads (1 mm diameter) at 37 °C, wherein the buffer comprises 33 mM PO₄ and 0.1% TWEEN20 (diamonds), 0.1% TritanX100 (squares), or 0.1% Pluoronic F-68 (triangles).

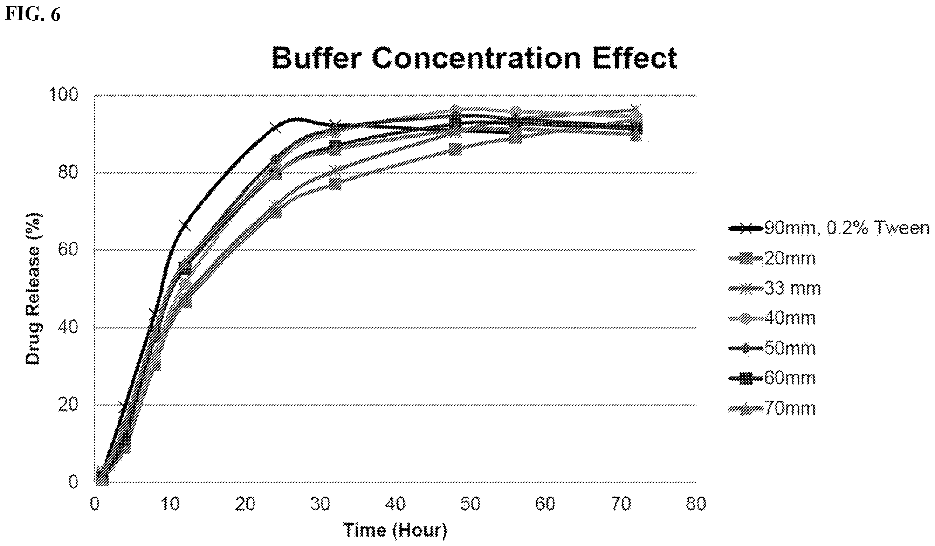

**FIG. 6** is a graphical representation of dissolution of drug product, exenatide, from LAI microspheres (y-axis) over time (x-axis), wherein the LAI is positioned under 4 g of glass beads (1 mm diameter) at 37 °C, wherein the buffer comprises 90 mM PO₄ and 0.2% TWEEN20 (x's), 20 mM PO₄ (squares), 33 mM PO₄ (stars), 40 mM PO₄ (circles), 50 mM PO₄ (diamonds), 60 mM PO₄ (dark squares), or 70 mM PO₄ (triangles).

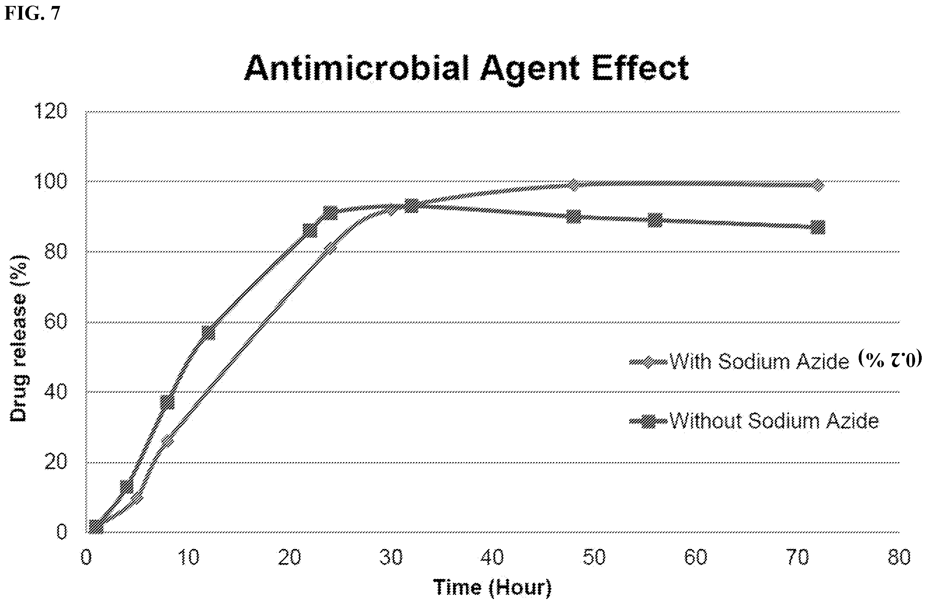

**FIG. 7** is a graphical representation of dissolution of drug product, exenatide, from LAI microspheres (y-axis) over time (x-axis), wherein the LAI is positioned under 4 g of glass beads (1 mm diameter) at 37 °C, wherein the buffer comprises 0.2% sodium azide (diamonds) or does not comprise sodium azide (squares).

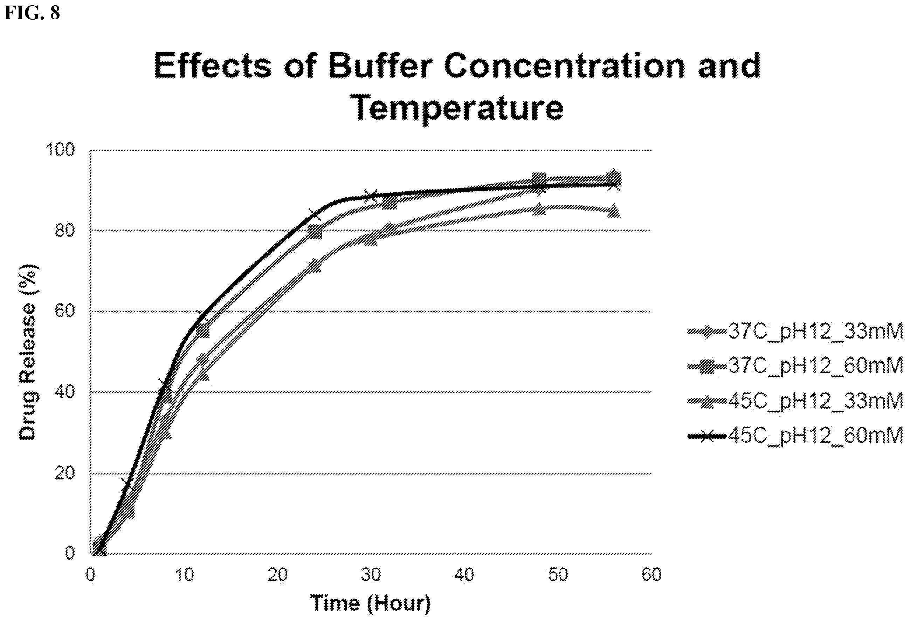

**FIG. 8** is a graphical representation of dissolution of drug product, exenatide, from LAI microspheres (y-axis) over time (x-axis), wherein the LAI is positioned under 4 g of glass beads (1 mm diameter) at pH 12 at (i) 37 °C in 33 mM PO₄ buffer (diamonds), (ii) 37 °C in 60 mM PO₄ buffer (squares), (iii) 45 °C in 33 mM PO₄ buffer (triangles), or (iv) 45 °C in 60 mM PO₄ buffer (x's).

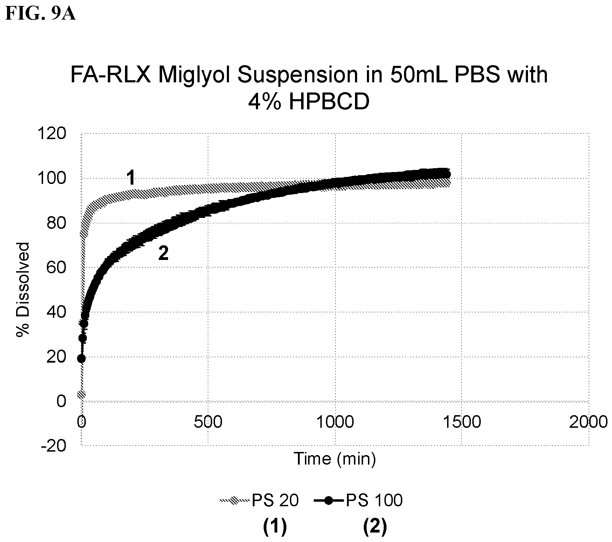

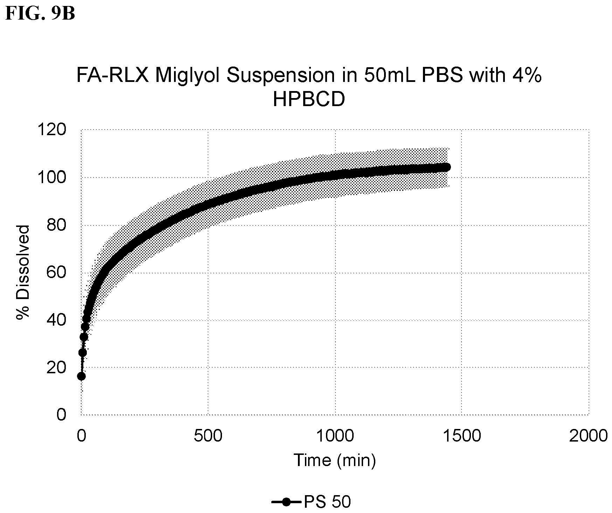

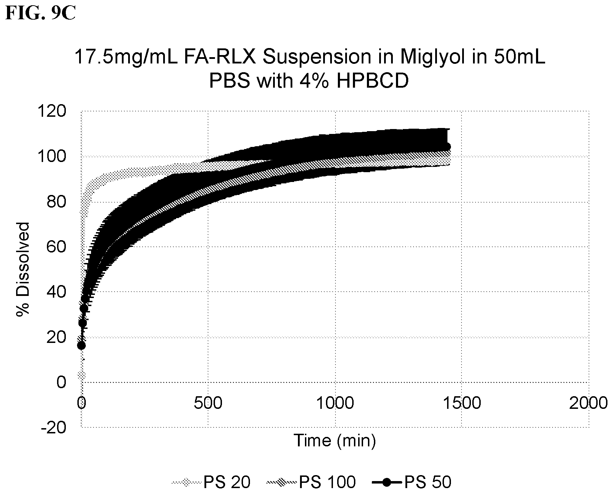

**FIGs. 9A-9C** are graphical representations of dissolution of a fatty acid-relaxin (FA-RLX) drug product at particle sizes (PS) of less than 20 (FIGs. 9A and 9C), less than 50 (FIGs. 9B-9C), and less than 100 µm (FIGs. 9A and 9C), wherein the FA-RLX is positioned under 0.5 mm glass beads and subjected to stirring at 35 rpm using a paddle. FIG. 9C presents an overlay of the data presented in FIGs. 9A and 9B.

### DETAILED DESCRIPTION OF THE DISCLOSURE

The present disclosure provides an *in vitro* dissolution method comprising agitating a solution placed in a vessel using a mobile paddle, wherein the solution comprises a solute, a media, and a plurality of beads. In some aspects, the plurality of beads is positioned between the solute and the mobile paddle and the mobile paddle is submerged in the solution. In some aspects, the solute is positioned on the bottom of the vessel. In some aspects, a first portion of the plurality of beads is positioned on the bottom of the vessel, the solute is positioned on top of the first portion of the plurality of beads, and a second portion of the plurality of beads is placed on top of the solute. The methods disclosed herein can be used for dissolution of any solute, including, but not limited to a long acting injectable (LAI), *e.g.*, an LAI microsphere loaded with a drug product.

### I. Terms

In order that the present disclosure can be more readily understood, certain terms are first defined. As used in this application, except as otherwise expressly provided herein, each of the following terms shall have the meaning set forth below. Additional definitions are set forth throughout the application.

It is to be noted that the term "a" or "an" entity refers to one or more of that entity; for example, "a nucleotide sequence," is understood to represent one or more nucleotide sequences. As such, the terms "a" (or "an"), "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

It is understood that wherever aspects are described herein with the language "comprising," otherwise analogous aspects described in terms of "consisting of" and/or "consisting essentially of" are also provided.

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

Units, prefixes, and symbols are denoted in their Système International de Unites (SI) accepted form. Numeric ranges are inclusive of the numbers defining the range. Unless otherwise indicated, nucleotide sequences are written left to right in 5' to 3' orientation. Amino acid sequences are written left to right in amino to carboxy orientation. The headings provided herein are not limitations of the various aspects of the disclosure, which can be had by reference to the specification as a whole. Accordingly, the terms defined immediately below are more fully defined by reference to the specification in its entirety.

The term "solute," as used herein, refers to any substance that is capable of dissolving in a solvent. In some aspects, the solute is in a solid state, *e.g.*, prior to or during dissolution using the methods disclosed herein. In some aspects, the solute is in a dissolved state, *e.g.*, following dissolution using the methods disclosed herein. In certain aspects, the solute is a long acting injectable (LAI). The methods disclosed herein can be used to dissolve any LAI. In some aspects, the LAI is a microsphere. In some aspects, the solute comprises an LAI microsphere loaded with a biologically active moiety.

The term "solvent," as used herein, refers to an liquid substance capable of dissolving a solute. In some aspects, the solvent comprises a buffer. In some aspects, the solvent comprises a potassium buffered solution, *e.g.*, PBS.

The term "long acting injectable" or "LAI," as used herein, refers to drug formulations that show an extended release profile, *e.g.*, over days or weeks. In some aspects, the LAI has a release profile of at least about 12 hours, at least about 18 hours, at least about 24 hours, at least about 36 hours, at least about 48 hours, at least about 60 hours, at least about 72 hours, at least about 84 hours, at least about 96 hours, at least about 108 hours, at least about 120 hours, at least about 6 days, at least about 7 days, at least about 8 days, at least about 9 days, at least about 10 days, at least about 11 days, at least about 12 days, at least about 13 days, at least about 14 days, at least about 15 days, at least about 16 days, at least about 17 days, at least about 18 days, at least about 19 days, at least about 20 days, or at least about 21 days, at least about 22 days. In some aspects, the LAI has a release profile of at least about 1 week, at least about 1.5 weeks, at least about 2 weeks, at least about 2.5 weeks, at least about 3 weeks, at least about 3.5 weeks, at least about 4 weeks, at least about 5 weeks, at least about 6 weeks, at least about 7 weeks, or at least about 8. In some aspects, the LAI comprises a biologically active moiety. In some aspects, the LAI comprises a biologically active moiety and a carrier. In some aspects, the carrier comprises a soluble microsphere. In some aspects, the microsphere is a poly-(D,L-lactide-Co-Glycolide) ("PLG") microsphere. The formula for PLG is shown as Formula I: 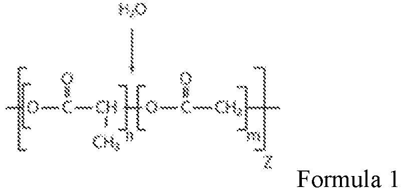 In some aspects, the LAI comprises hydrogel. In some aspects, the LAI comprises a liquid crystal. In some aspects, the LAI comprises a combination of one or more of a microsphere, hydrogel, and a liquid crystal. In some aspects, the biologically active moiety is encapsulated within the LAI, *e.g.*, within the PLG microsphere.

A "biologically active moiety," as used herein, refers to any substance which can affect any physical or biochemical properties of a biological system, pathway, molecule, or interaction relating to a living organism. In particular, as used herein, biologically active molecules include, but are not limited to, any substance intended for diagnosis, cure, mitigation, treatment, or prevention of disease or conditions, e.g., diseases or conditions associated with fibrosis, in humans or other animals, or to otherwise enhance physical or mental well-being of humans or animals. Biologically active moieties can comprise a polypeptide (*e.g.*, a protein (*e.g.*, an enzyme, a growth factor, a cytokine, a chemokine, a ligand, a receptor, a hormone, an antibody, an antigen, a fragment thereof, or any combination thereof), a nucleotide (*e.g.*, a DNA (*e.g.*, a ssDNA, a dsDNA, a plasmid, a fragment thereof, or any combination thereof) an RNA (*e.g.*, a mRNA, a miRNA, an siRNA, a dsRNA, a fragment thereof, or any combination thereof), an antisense oligomer, a fragment thereof, or any combination thereof), a Cas9 nuclease, a TALEN nuclease, a zinc finger nuclease, a small molecule, or any combination thereof. In some aspects, the biologically active moiety comprises a small molecule. In some aspects, the biologically active moiety comprise a polypeptide. In some aspects, the polypeptide has a molecular weight of a molecular weight of less than about 20 kDa, less than about 15 kDa, less than about 14 kDa, less than about 13 kDa, less than about 12 kDa, less than about 11 kDa, less than about 10 kDa, less than about 9 kDa, less than about 8 kDa, less than about 7 kDa, less than about 6 kDa, less than about 5 kDa, less than about 4 kDa. In some aspects, the polypeptide has a molecular weight of about 4 kDa, about 5 kDa, about 6 kDa, about 7 kDa, about 8 kDa, about 9 kDa, or about 10 kDa.

A "therapeutically effective amount" or "therapeutically effective dosage" of a drug or therapeutic agent is any amount of the drug that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression can be evaluated using a variety of methods known to the skilled practitioner, such as in human subjects during clinical trials, in animal model systems predictive of efficacy in humans, or by assaying the activity of the agent in *in vitro* assays. In some aspects, the methods described herein are used to measure the solubility of a therapeutically effective amount of a LAI drug product *in vitro.*

A "subject" includes any human or nonhuman animal. The term "nonhuman animal" includes, but is not limited to, vertebrates such as nonhuman primates, sheep, dogs, and rodents such as mice, rats and guinea pigs. In preferred aspects, the subject is a human. The terms, "subject" and "patient" are used interchangeably herein.

The terms "about" or "comprising essentially of" refer to a value or composition that is within an acceptable error range for the particular value or composition as determined by one of ordinary skill in the art, which will depend in part on how the value or composition is measured or determined, *i.e.*, the limitations of the measurement system. For example, "about" or "comprising essentially of" can mean within 1 or more than 1 standard deviation per the practice in the art. Alternatively, "about" or "comprising essentially of" can mean a range of up to 10%. Furthermore, particularly with respect to biological systems or processes, the terms can mean up to an order of magnitude or up to 5-fold of a value. When particular values or compositions are provided in the application and claims, unless otherwise stated, the meaning of "about" or "comprising essentially of" should be assumed to be within an acceptable error range for that particular value or composition.

As described herein, any concentration range, percentage range, ratio range or integer range is to be understood to include the value of any integer within the recited range and, when appropriate, fractions thereof (such as one tenth and one hundredth of an integer), unless otherwise indicated.

Various aspects of the disclosure are described in further detail in the following subsections.

### II. Methods of the Disclosure

Certain aspects of the present disclosure are directed to an *in vitro* dissolution method comprising agitating a solution placed in a vessel using a mobile paddle; wherein the solution comprises a solute, a media, and a plurality of beads; and wherein the paddle is submerged in the solution. In some aspects, the plurality of beads is positioned between the solute and the mobile paddle.

The methods disclosed herein provide improved dissolution and solution quality over existing methods. In particular, the most common methods *in vitro* dissolution of LAI are (1) sample and separate methods, (2) dialysis methods, and (3) flow-through methods. *See, e.g.*, Seidlitz et al., J. Pharm. Pharmacol. 64:969-85 (2012). Though these methods have been applied successfully to small molecule LAIs, they have been less successful in dissolution of formulations comprising large molecules such as peptides, for example leading to peptide aggregation or particle loss. The dialysis method is subject to molecular mass cut-off value of dialysis membrane that may affect the peptide diffusion to bulk after released from LAI formulation. The flow-through method uses compendial USP apparatus 4 ("USP IV"), but suffers from less effective sterilization when being used for peptide LAI and causes the peptide degradation during dissolution testing.

The methods disclosed herein overcome the limitations of conventional methods by placing the solute in the vessel beneath a plurality of beads, *e.g.*, glass beads, the submerging the solute and the beads in a media, wherein a mobile paddle is submerged in the media and positioned above the beads, such that when the mobile paddle is in a stationary position, the mobile paddle is not in contact with the beads. These methods provide for increased consistency, robustness, and dissolution over a shorter period of time than conventional methods.

In some aspects, the method comprises, prior to the agitating, loading the solution into the vessel by (i) adding the solute to the bottom of the vessel; (ii) adding the plurality of beads to the bottom of the vessel, wherein the plurality of beads are added on top of the solute; and (iii) adding a volume of media to the vessel, wherein the media is added on top of the solute and the plurality of beads.

Any vessel can be used in the methods disclosed herein. In some aspects, the vessel is a vile of sufficient size to enclose at least about 100 mL of solution and a mobile paddle, wherein the mobile paddle is submerged in the at least about 100 mL of solution. In some aspects, the vessel is a small volume vessel. In some aspects, the vessel is a HANSON small volume vessel. In some aspects, the vessel is placed in a dissolution bath during the dissolution. Any dissolution bath can be used in the methods disclosed herein. In some aspects, the dissolution bath maintains a temperature of at least about 34 °C, at least about 35 °C, at least about 36 °C, at least about 37 °C, at least about 38 °C, at least about 39 °C, at least about 40 °C, at least about 41 °C, at least about 42 °C, at least about 43 °C, at least about 44 °C, or at least about 45 °C. In some aspects, the dissolution bath maintains a temperature of about 37 °C. In some aspects, the dissolution bath maintains a temperature of about 38 °C. In some aspects, the dissolution bath maintains a temperature of about 39°C. In some aspects, the dissolution bath maintains a temperature of about 40°C. In some aspects, the dissolution bath maintains a temperature of about 41°C. In some aspects, the dissolution bath maintains a temperature of about 42°C. In some aspects, the dissolution bath maintains a temperature of about 43°C. In some aspects, the dissolution bath maintains a temperature of about 44°C. In some aspects, the dissolution bath maintains a temperature of about 45 °C.

In some aspects, the solute is added directly to the bottom of the vessel. In some aspects, a first plurality of beads is added to the bottom of the vessel, and then the solute is added on top of the first plurality of beads. Any volume of solute can be subjected to the methods disclosed herein. Increasing the amount of solute can be matched by increase one or more of the size of the vessel, the amount of the plurality of beads, and/or the volume of the solvent added to the vessel. In some aspects, the amount of solute is equivalent to a single unit dose, *e.g.*, a therapeutically effective dose, of the drug product. In some aspects, the amount of the solute is intended to match the therapeutically effective dose of the drug product so that the method can be used to accurately predict *in vivo* dissolution of the drug product.

In some aspects, a vehicle solution is added on top of the solute, *e.g.*, to wet the solute. In some aspects, the vehicle solution comprises a solution used in the storage of the solute, *e.g.*, the drug product formulation. In some aspects, the volume of the vehicle solution is sufficient to wet but not completely submerge the solute and/or the plurality of beads. In some aspects, less than about 5 mL, less than about 4.5 mL, less than about 4 mL, less than about 3.5 mL, less than about 3 mL, less than about 2.5 mL, less than about 2 mL, less than about 1.5 mL, less than about 1 mL, less than about 0.5 mL of vehicle solution is added on top of the solute. In some aspects, about 0.5 mL of the vehicle solution is added to a therapeutically effective amount of the LAI.

In some aspects, a plurality of beads is added on top of the solute in the vessel. Any beads can be used in the methods disclosed herein. In some aspects, the plurality of beads comprises one or more glass beads, one or more plastic beads, one or more silicate beads, one or more metal beads, or any combination thereof. In some aspects, the plurality of beads comprises glass beads. In some aspects, the plurality of beads comprises plastic beads. In some aspects, the plurality of beads comprises silicate beads. In some aspects, the plurality of beads comprises metal beads.

Beads of any size can be used in the methods disclosed herein. In some aspects, the plurality of beads comprises one or more beads having a diameter of at least about 0.01 mm, at least about 0.02 mm, at least about 0.03 mm, at least about 0.04 mm, at least about 0.05 mm, at least about 0.06 mm, at least about 0.07 mm, at least about 0.08 mm, at least about 0.09 mm, at least about 0.1 mm, at least about 0.2 mm, at least about 0.3 mm, at least about 0.4 mm, at least about 0.5 mm, at least about 0.6 mm, at least about 0.7 mm, at least about 0.8 mm, at least about 0.9 mm, at least about 1.0 mm, at least about 1.1 mm, at least about 1.2 mm, at least about 1.3 mm, at least about 1.4 mm, at least about 1.5 mm, at least about 1.6 mm, at least about 1.7 mm, at least about 1.8 mm, at least about 1.9 mm, or at least about 2.0 mm. In some aspects, the plurality of beads comprises one or more beads having a diameter of about 0.5 mm. In some aspects, the plurality of beads comprises one or more beads having a diameter of about 0.6 mm. In some aspects, the plurality of beads comprises one or more beads having a diameter of about 0.7 mm. In some aspects, the plurality of beads comprises one or more beads having a diameter of about 0.8 mm. In some aspects, the plurality of beads comprises one or more beads having a diameter of about 0.9 mm. In some aspects, the plurality of beads comprises one or more beads having a diameter of about 1.0 mm. In some aspects, the plurality of beads comprises one or more beads having a diameter of about 1.1 mm. In some aspects, the plurality of beads comprises one or more beads having a diameter of about 1.2 mm. In some aspects, the plurality of beads comprises one or more beads having a diameter of about 1.3 mm. In some aspects, the plurality of beads comprises one or more beads having a diameter of about 1.4 mm. In some aspects, the plurality of beads comprises one or more beads having a diameter of about 1.5 mm.

In some aspects, the plurality of beads comprises beads of equal size. In some aspects, the plurality of beads comprises a mixture of beads of different sizes. In some aspects, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% of the beads are of approximately the same size (*e.g.*, within a range of about 0.8 mm to about 1.2 mm). In some aspects, at least about 50% of the beads are of approximately the same size (*e.g.*, within a range of about 0.8 mm to about 1.2 mm). In some aspects, at least about 60% of the beads are of approximately the same size (*e.g.*, within a range of about 0.8 mm to about 1.2 mm). In some aspects, at least about 70% of the beads are of approximately the same size (*e.g.*, within a range of about 0.8 mm to about 1.2 mm). In some aspects, at least about 75% of the beads are of approximately the same size (*e.g.*, within a range of about 0.8 mm to about 1.2 mm). In some aspects, at least about 80% of the beads are of approximately the same size (*e.g.*, within a range of about 0.8 mm to about 1.2 mm). In some aspects, at least about 85% of the beads are of approximately the same size (*e.g.*, within a range of about 0.8 mm to about 1.2 mm). In some aspects, at least about 90% of the beads are of approximately the same size (*e.g.*, within a range of about 0.8 mm to about 1.2 mm). In some aspects, at least about 95% of the beads are of approximately the same size (*e.g.*, within a range of about 0.8 mm to about 1.2 mm). In some aspects, at least about 96% of the beads are of approximately the same size (*e.g.*, within a range of about 0.8 mm to about 1.2 mm). In some aspects, at least about 97% of the beads are of approximately the same size (*e.g.*, within a range of about 0.8 mm to about 1.2 mm). In some aspects, at least about 98% of the beads are of approximately the same size (*e.g.*, within a range of about 0.8 mm to about 1.2 mm). In some aspects, at least about 99% of the beads are of approximately the same size (*e.g.*, within a range of about 0.8 mm to about 1.2 mm).

The amount of the plurality of beads added to the solute can be adjusted based on the amount of solute, the volume of the vessel, the shape of the vessel, the volume of the solution, or any combination thereof. In some aspects, the amount of beads in the plurality of beads is equivalent to the amount necessary to cover the solute in beads. In some aspects, the solute is positioned below the beads. In some aspects, the solute is sandwiched by the beads, *e.g.*, the solute is positioned such that there are beads both on top of and below the solute. In some aspects, the plurality of beads comprises a volume equivalent to 2 g of glass beads, wherein the average diameter of the glass beads is about 1.0 mm). For example, in aspects where the plurality of beads comprises plastic beads, the volume of plastic beads could weigh less than the equivalent volume of glass beads, *i.e.*, the equivalent of 3 g of glass beads may be 2 g of plastic beads. Conversely, metal beads may have a higher unit mass than similarly sized glass beads. As such, in aspects where the plurality of beads comprises metal beads, the equivalent of 3 g of glass beads may be 4 g of metal beads. In some aspects, the plurality of beads comprises a volume equivalent to 2.5 g of glass beads, wherein the average diameter of the glass beads is about 1.0 mm). In some aspects, the plurality of beads comprises a volume equivalent to 3 g of glass beads, wherein the average diameter of the glass beads is about 1.0 mm). In some aspects, the plurality of beads comprises a volume equivalent to 3.1 g of glass beads, wherein the average diameter of the glass beads is about 1.0 mm). In some aspects, the plurality of beads comprises a volume equivalent to 3.2 g of glass beads, wherein the average diameter of the glass beads is about 1.0 mm). In some aspects, the plurality of beads comprises a volume equivalent to 3.3 g of glass beads, wherein the average diameter of the glass beads is about 1.0 mm). In some aspects, the plurality of beads comprises a volume equivalent to 3.4 g of glass beads, wherein the average diameter of the glass beads is about 1.0 mm). In some aspects, the plurality of beads comprises a volume equivalent to 3.5 g of glass beads, wherein the average diameter of the glass beads is about 1.0 mm). In some aspects, the plurality of beads comprises a volume equivalent to 3.6 g of glass beads, wherein the average diameter of the glass beads is about 1.0 mm). In some aspects, the plurality of beads comprises a volume equivalent to 3.7 g of glass beads, wherein the average diameter of the glass beads is about 1.0 mm). In some aspects, the plurality of beads comprises a volume equivalent to 3.8 g of glass beads, wherein the average diameter of the glass beads is about 1.0 mm). In some aspects, the plurality of beads comprises a volume equivalent to 3.9 g of glass beads, wherein the average diameter of the glass beads is about 1.0 mm). In some aspects, the plurality of beads comprises a volume equivalent to 4.0 g of glass beads, wherein the average diameter of the glass beads is about 1.0 mm). In some aspects, the plurality of beads comprises a volume equivalent to 4.1 g of glass beads, wherein the average diameter of the glass beads is about 1.0 mm). In some aspects, the plurality of beads comprises a volume equivalent to 4.2 g of glass beads, wherein the average diameter of the glass beads is about 1.0 mm). In some aspects, the plurality of beads comprises a volume equivalent to 4.3 g of glass beads, wherein the average diameter of the glass beads is about 1.0 mm). In some aspects, the plurality of beads comprises a volume equivalent to 4.4 g of glass beads, wherein the average diameter of the glass beads is about 1.0 mm). In some aspects, the plurality of beads comprises a volume equivalent to 4.5 g of glass beads, wherein the average diameter of the glass beads is about 1.0 mm). In some aspects, the plurality of beads comprises a volume equivalent to 4.6 g of glass beads, wherein the average diameter of the glass beads is about 1.0 mm). In some aspects, the plurality of beads comprises a volume equivalent to 4.7 g of glass beads, wherein the average diameter of the glass beads is about 1.0 mm). In some aspects, the plurality of beads comprises a volume equivalent to 4.8 g of glass beads, wherein the average diameter of the glass beads is about 1.0 mm). In some aspects, the plurality of beads comprises a volume equivalent to 4.9 g of glass beads, wherein the average diameter of the glass beads is about 1.0 mm). In some aspects, the plurality of beads comprises a volume equivalent to 5.0 g of glass beads, wherein the average diameter of the glass beads is about 1.0 mm). In some aspects, the plurality of beads comprises a volume equivalent to 5.5 g of glass beads, wherein the average diameter of the glass beads is about 1.0 mm). In some aspects, the plurality of beads comprises a volume equivalent to 6.0 g of glass beads, wherein the average diameter of the glass beads is about 1.0 mm). In some aspects, the plurality of beads comprises a volume equivalent to 6.5 g of glass beads, wherein the average diameter of the glass beads is about 1.0 mm). In some aspects, the plurality of beads comprises a volume equivalent to 7.0 g of glass beads, wherein the average diameter of the glass beads is about 1.0 mm). In some aspects, the plurality of beads comprises a volume equivalent to 7.5 g of glass beads, wherein the average diameter of the glass beads is about 1.0 mm). In some aspects, the plurality of beads comprises a volume equivalent to 8.0 g of glass beads, wherein the average diameter of the glass beads is about 1.0 mm).

In some aspects, the plurality of beads comprises at least about 2 g of beads (*e.g.*, glass beads of approximately 1 mm in diameter) per about 100 mL of solution, at least about 2.5 g of beads (*e.g.*, glass beads of approximately 1 mm in diameter) per about 100 mL of solution, at least about 3 g of beads (*e.g.*, glass beads of approximately 1 mm in diameter) per about 100 mL of solution, at least about 3.1 g of beads (*e.g.*, glass beads of approximately 1 mm in diameter) per about 100 mL of solution, at least about 3.2 g of beads (*e.g.*, glass beads of approximately 1 mm in diameter) per about 100 mL of solution, at least about 3.3 g of beads (*e.g.*, glass beads of approximately 1 mm in diameter) per about 100 mL of solution, at least about 3.4 g of beads (*e.g.*, glass beads of approximately 1 mm in diameter) per about 100 mL of solution, at least about 3.5 g of beads (*e.g.*, glass beads of approximately 1 mm in diameter) per about 100 mL of solution, at least about 3.6 g of beads (*e.g.*, glass beads of approximately 1 mm in diameter) per about 100 mL of solution, at least about 3.7 g of beads (*e.g.*, glass beads of approximately 1 mm in diameter) per about 100 mL of solution, at least about 3.8 g of beads (*e.g.*, glass beads of approximately 1 mm in diameter) per about 100 mL of solution, at least about 3.9 g of beads (*e.g.*, glass beads of approximately 1 mm in diameter) per about 100 mL of solution, at least about 4 g of beads (*e.g.*, glass beads of approximately 1 mm in diameter) per 100 mL of about solution, at least about 4.1 g of beads (*e.g.*, glass beads of approximately 1 mm in diameter) per 100 mL of about solution, at least about 4.2 g of beads (*e.g.*, glass beads of approximately 1 mm in diameter) per 100 mL of about solution, at least about 4.3 g of beads (*e.g.*, glass beads of approximately 1 mm in diameter) per 100 mL of about solution, at least about 4.4 g of beads (*e.g.*, glass beads of approximately 1 mm in diameter) per 100 mL of about solution, at least about 4.5 g of beads (*e.g.*, glass beads of approximately 1 mm in diameter) per 100 mL of about solution, at least about 4.6 g of beads (*e.g.*, glass beads of approximately 1 mm in diameter) per 100 mL of about solution, at least about 4.7 g of beads (*e.g.*, glass beads of approximately 1 mm in diameter) per 100 mL of about solution, at least about 4.8 g of beads (*e.g.*, glass beads of approximately 1 mm in diameter) per 100 mL of about solution, at least about 4.9 g of beads (*e.g.*, glass beads of approximately 1 mm in diameter) per 100 mL of about solution, at least about 5 g of beads (*e.g.*, glass beads of approximately 1 mm in diameter) per about 100 mL of solution, at least about 6 g of beads per about 100 mL of solution, or at least about 7 g of beads (*e.g.*, glass beads of approximately 1 mm in diameter) per about 100 mL of solution. In some aspects, the plurality of beads comprises about 3.5 g of beads (*e.g.*, glass beads of approximately 1 mm in diameter) per about 100 mL of solution. In some aspects, the plurality of beads comprises about 3.6 g of beads (*e.g.*, glass beads of approximately 1 mm in diameter) per about 100 mL of solution. In some aspects, the plurality of beads comprises about 3.7 g of beads (*e.g.*, glass beads of approximately 1 mm in diameter) per about 100 mL of solution. In some aspects, the plurality of beads comprises about 3.8 g of beads (*e.g.*, glass beads of approximately 1 mm in diameter) per about 100 mL of solution. In some aspects, the plurality of beads comprises about 3.9 g of beads (*e.g.*, glass beads of approximately 1 mm in diameter) per about 100 mL of solution. In some aspects, the plurality of beads comprises about 4 g of beads (*e.g.*, glass beads of approximately 1 mm in diameter) per 100 mL of about solution. In some aspects, the plurality of beads comprises about 4.1 g of beads (*e.g.*, glass beads of approximately 1 mm in diameter) per 100 mL of about solution. In some aspects, the plurality of beads comprises about 4.2 g of beads (*e.g.*, glass beads of approximately 1 mm in diameter) per 100 mL of about solution. In some aspects, the plurality of beads comprises about 4.3 g of beads (*e.g.*, glass beads of approximately 1 mm in diameter) per 100 mL of about solution. In some aspects, the plurality of beads comprises about 4.4 g of beads (*e.g.*, glass beads of approximately 1 mm in diameter) per 100 mL of about solution. In some aspects, the plurality of beads comprises about 4.5 g of beads (*e.g.*, glass beads of approximately 1 mm in diameter) per 100 mL of about solution. In some aspects, the plurality of beads comprises about 5 g of beads (*e.g.*, glass beads of approximately 1 mm in diameter) per 100 mL of about solution. In some aspects, the plurality of beads comprises about 5.5 g of beads (*e.g.*, glass beads of approximately 1 mm in diameter) per 100 mL of about solution. In some aspects, the plurality of beads comprises about 6 g of beads (*e.g.*, glass beads of approximately 1 mm in diameter) per 100 mL of about solution. In some aspects, the plurality of beads comprises about 6.5 g of beads (*e.g.*, glass beads of approximately 1 mm in diameter) per 100 mL of about solution. In some aspects, the plurality of beads comprises about 7 g of beads (*e.g.*, glass beads of approximately 1 mm in diameter) per 100 mL of about solution. In some aspects, the plurality of beads comprises about 7.5 g of beads (*e.g.*, glass beads of approximately 1 mm in diameter) per 100 mL of about solution.

In some aspects, the vessel is filled with a media, *e.g.*, a solvent and/or a buffer, following addition of the plurality of beads. Any media can be used in the methods disclosed herein. In some aspects, the media is tailored to the solute. In some aspects, the media comprises a phosphate buffer. In some aspects, the media comprises phosphate buffered saline (PBS). In some aspects, the media comprises at least about 20 mM PO₄, at least about 25 mM PO₄, at least about 30 mM PO₄, at least about 33 mM PO₄, at least about 35 mM PO₄, at least about 40 mM PO₄, at least about 45 mM PO₄, at least about 50 mM PO₄, at least about 55 mM PO₄, at least about 60 mM PO₄, at least about 65 mM PO₄, at least about 70 mM PO₄, at least about 75 mM PO₄, at least about 80 mM PO₄, at least about 90 mM PO₄, at least about 95 mM PO₄, or at least about 100 mM PO₄. In some aspects, the media comprises at least about 33 mM PO₄. In some aspects, the media comprises at least about 40 mM PO₄. In some aspects, the media comprises at least about 50 mM PO₄. In some aspects, the media comprises at least about 60 mM PO₄. In some aspects, the media comprises at least about 70 mM PO₄. In some aspects, the media comprises at least about 80 mM PO₄. In some aspects, the media comprises at least about 90 mM PO₄. In some aspects, the media comprises at least about 100 mM PO₄.

In some aspects, the media further comprises a surfactant. In some aspects, the surfactant is selected from polysorbate 20 (TWEEN 20), polysorbate 80 (TWEEN 80), TritonX100, pluoronic F-68, and any combination thereof. In some aspects, the surfactant comprises polysorbate 20 (TWEEN 20). In some aspects, the surfactant comprises polysorbate 80 (TWEEN 80). In some aspects, the surfactant comprises TritonX100. In some aspects, the surfactant comprises pluoronic F-68. In some aspects, the media comprises at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.8%, at least about 0.9%, at least about 1.0% surfactant (*e.g.*, polysorbate 20). In some aspects, the media comprises about 0.1% surfactant (*e.g.*, polysorbate 20). In some aspects, the media comprises about 0.2% surfactant (*e.g.*, polysorbate 20). In some aspects, the media comprises about 0.3% surfactant (*e.g.*, polysorbate 20). In some aspects, the media comprises about 0.4% surfactant (*e.g.*, polysorbate 20). In some aspects, the media comprises about 0.5% surfactant (*e.g.*, polysorbate 20).

In some aspects, the media further comprises an antimicrobial agent. Any antimicrobial agent can be used in the methods disclosed herein. In some aspects, the microbial agent comprises sodium azide. In some aspects, the media comprises at least about 0.01%, at least about 0.02%, at least about 0.03%, at least about 0.04%, at least about 0.05%, at least about 0.06%, at least about 0.07%, at least about 0.08%, at least about 0.09%, at least about 0.1%, at least about 0.2%, at least about 0.3%, at least about 0.4%, at least about 0.5%, at least about 0.6%, at least about 0.7%, at least about 0.8%, at least about 0.9%, at least about 1.0% antimicrobial agent (e.g., sodium azide). In some aspects, the media comprises about 0.1% antimicrobial agent (e.g., sodium azide). In some aspects, the media comprises about 0.2% antimicrobial agent (e.g., sodium azide). In some aspects, the media comprises about 0.3% antimicrobial agent (e.g., sodium azide). In some aspects, the media comprises about 0.4% antimicrobial agent (e.g., sodium azide). In some aspects, the media comprises about 0.5% antimicrobial agent (e.g., sodium azide).

In some aspects, the media is added on top of the plurality of beads, which is positioned on top of the solute. In some aspects, the media is added at a rate that does not cause agitation of the solute and/or the plurality of beads. In some aspects, the volume of the solution in the vessel is at least about 50 mL, at least about 60 mL, at least about 70 mL, at least about 75 mL, at least about 80 mL, at least about 90 mL, or at least about 100 mL. In some aspects, volume of the solution in the vessel is about 90 mL. In some aspects, volume of the solution in the vessel is about 100 mL. In some aspects, volume of the solution in the vessel is about 110 mL. In some aspects, volume of the solution in the vessel is about 120 mL. In some aspects, volume of the solution in the vessel is about 130 mL. In some aspects, volume of the solution in the vessel is about 140 mL. In some aspects, volume of the solution in the vessel is about 150 mL. In some aspects, volume of the solution in the vessel is about 160 mL. In some aspects, volume of the solution in the vessel is about 170 mL. In some aspects, volume of the solution in the vessel is about 180 mL. In some aspects, volume of the solution in the vessel is about 190 mL. In some aspects, volume of the solution in the vessel is about 200 mL. In some aspects, volume of the solution in the vessel is about 300 mL. In some aspects, volume of the solution in the vessel is about 400 mL. In some aspects, volume of the solution in the vessel is about 500 mL. The media is added while the mobile paddle is in a stationary position. The total volume of the solution will depend on the total amount of solute being subjected to the methods disclosed herein. A higher amount of solute will require a higher volume of total solution. The total volume of solution must be sufficient to submerge the solute, the plurality of beads, and the mobile paddle.

Any mobile paddle can be used in the methods disclosed herein. In some aspects, the mobile paddle is mounted to the vessel, such that the mobile paddle is positioned within the vessel below the solution surface and above the plurality of beads. In some aspects, the mobile paddle is not mounted directly to the vessel, but is positioned such that the mobile paddle is in the vessel below the solution surface and above the plurality of beads. In some aspects, the size of the paddle is such that the paddle does not touch the walls of the vessel. A mobile paddle of the present disclosure is capable of moving, *e.g.*, spinning, in such a way as to agitate the solution. In some aspects, after the solute, the plurality of beads, and the media are added to the vessel, the method further comprises applying a force to the mobile paddle that causes the mobile paddle to spin and/or oscillate, thereby agitating the solution. In some aspects, the mobile paddle spins at a rate of at least about 10 rpm, at least about 15 rpm, at least about 20 rpm, at least about 25 rpm, at least about 30 rpm, at least about 35 rpm, at least about 40 rpm, at least about 45 rpm, at least about 50 rpm, at least about 55 rpm, at least about 60 rpm, at least about 65 rpm, at least about 70 rpm, at least about 75 rpm, at least about 80 rpm, at least about 85 rpm, at least about 90 rpm, at least about 95 rpm, at least about 100 rpm, at least about 105 rpm, at least about 110 rpm, at least about 115 rpm, at least about 120 rpm, at least about 125 rpm, at least about 130 rpm, at least about 135 rpm, at least about 140 rpm, at least about 145 rpm, at least about 150 rpm, at least about 160 rpm, at least about 170 rpm, at least about 180 rpm, at least about 190 rpm, or at least about 200 rpm. In some aspects, the mobile paddle spins at a rate of at least about 15 rpm. In some aspects, the mobile paddle spins at a rate of at least about 20 rpm. In some aspects, the mobile paddle spins at a rate of at least about 25 rpm. In some aspects, the mobile paddle spins at a rate of at least about 30 rpm. In some aspects, the mobile paddle spins at a rate of at least about 35 rpm. In some aspects, the mobile paddle spins at a rate of at least about 40 rpm. In some aspects, the mobile paddle spins at a rate of at least about 45 rpm. In some aspects, the mobile paddle spins at a rate of at least about 50 rpm. In some aspects, the mobile paddle spins at a rate of at least about 55 rpm. In some aspects, the mobile paddle spins at a rate of at least about 60 rpm. In some aspects, the mobile paddle spins at a rate of at least about 65 rpm. In some aspects, the mobile paddle spins at a rate of at least about 70 rpm. In some aspects, the mobile paddle spins at a rate of at least about 75 rpm. In some aspects, the mobile paddle spins at a rate of at least about 80 rpm. In some aspects, the mobile paddle spins at a rate of at least about 85 rpm. In some aspects, the mobile paddle spins at a rate of at least about 90 rpm. In some aspects, the mobile paddle spins at a rate of at least about 95 rpm. In some aspects, the mobile paddle spins at a rate of at least about 100 rpm. In some aspects, the mobile paddle spins at a rate of at least about 105 rpm. In some aspects, the mobile paddle spins at a rate of at least about 110 rpm. In some aspects, the mobile paddle spins at a rate of at least about 115 rpm. In some aspects, the mobile paddle spins at a rate of at least about 120 rpm. In some aspects, the mobile paddle spins at a rate of at least about 125 rpm.

The duration of the dissolution methods disclosed herein can depend on the solute. In some aspects, the dissolution method is applied to the solute for a sufficient amount of time for at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, at least about 99%, or about 100% of the solute to dissolve in the media. In some aspects, the dissolution method is applied to the solute for at least about 6 hours, at least about 12 hours, at least about 18 hours, at least about 24 hours, at least about 30 hours, at least about 36 hours, at least about 42 hours, at least about 48 hours, at least about 60 hours, at least about 72 hours, at least about 84 hours, or at least about 96 hours.

Dissolution of the solute can be measured using any methods. In some aspects, a probe is inserted into the vessel, below the level of the solution. In some aspects, the probe is placed above the mobile paddle. In some aspects, the probe comprises UV fiber optics for in-situ measurement. In some aspects, a sample is removed from the solution during the dissolution process, and the concentration of the solute in the sample is measured. In some aspects, the sample is obtained from the solution at a position above the mobile paddle. In some aspects, after the sample is obtained, a volume of media is added to the solution that is equivalent to the volume of the sample removed from the solution.

In some aspects, the vessel is covered during the dissolution process. In some aspects, the vessel is covered during the dissolution process, wherein the cover is only removed during sample collection.

In some aspects, the method comprises: (i) adding a solute to the bottom of a vessel, wherein the solute comprises a long acting injectable microsphere; (ii) adding a plurality of beads on top of the solute, wherein the plurality of beads comprises one or more glass beads; (iii) adding a media to the solute and the volume of beads, wherein the media is added without agitating the solute or the volume of beads, and wherein the media is added at a volume that is sufficient to submerge the mobile paddle; and applying a force to cause the mobile paddle to spin and/or oscillate, agitating the solution. In some aspects, the agitation is applied until at least about 80% of the solute is dissolved in the solution. In some aspects, the agitation is applied until about 100% of the solute is dissolved in the solution.

In some aspects, the method comprises (i) adding a first plurality of beads to the bottom of a vessel, wherein the plurality of beads comprises one or more glass beads; (ii) adding a solute on top of the plurality of beads, wherein the solute comprises a long acting injectable microsphere; (iii) adding a second plurality of beads on top of the solute, wherein the plurality of beads comprises one or more glass beads; (iv) adding a media to the solute and the volume of beads, wherein the media is added without agitating the solute or the volume of beads, and wherein the media is added at a volume that is sufficient to submerge the mobile paddle; and (v) applying a force to cause the mobile paddle to spin and/or oscillate, agitating the solution. In some aspects, the agitation is applied until at least about 80% of the solute is dissolved in the solution. In some aspects, the agitation is applied until about 100% of the solute is dissolved in the solution.

Certain aspects of the present disclosure are directed to a kit comprising (i) a vessel; (ii) a plurality of beads; (iii) a media; (iv) a mobile paddle, wherein the mobile paddle can be positioned inside the vessel; and (v) instructions for dissolving a solute according to any method disclosed herein. Certain aspects of the present disclosure are directed to a kit comprising (i) a vessel; (ii) a plurality of beads; (iii) a media; (iv) a mobile paddle, wherein the mobile paddle can be positioned inside the vessel; (v) a cover for the vessel; and (vi) instructions for dissolving a solute according to any method disclosed herein.

The following examples are offered by way of illustration and not by way of limitation.

### EXAMPLES

### Example 1

A novel setting for dissolution of LAIs was developed wherein an LAI sample is sandwiched by glass beads at the bottom of a vessel, and a paddle over the glass beads for agitation (FIG. 1). The present example demonstrates the application of the method to LAIs (a PLGA microsphere formulation and a hydrogel formulation) containing peptide drugs. The molecular weight of the peptide used in the present example is approximately 4 kDa. The microsphere drug loading is 5% w/w for 2 mg API/dose, designed for weekly injection.

Small volume vessels (Hanson) on a Distek 2500 dissolution bath were used, with 100 mL medium in each vessel held at 37 °C. A mini paddle was used at 100 rpm. A single dose equivalent amount of the microsphere formulation was placed in the vessel first, followed by adding 0.5 mL vehicle to wet the microsphere. Glass beads (1 mm diameter) were added on top of the sample, followed by carefully adding 100 mL of media without disturbing the glass beads.

The following method parameters were evaluated: (1) amount of glass beads (3g, 4g, 6g, and 9g); (2) phosphate buffer pH (7.4, 9.8, 11, and 12); (3) buffer concentration (20, 33, 40, 50, 60, 70, and 90 mM); (4) surfactant (TWEEN20, Triton X100, and Pluoronic F-68); (5) surfactant concentration (0.1%, 0.2%, and 0.3%); (6) temperature of media (37°C and 45°C); (7) antimicrobial agent (with and without); and (8) evaporation control (with and without).

The drug release was tested by withdrawing 1 mL of aliquot from the bulk with refill of equal amount of fresh media at predetermined time points (1, 2, 4, 8, 12, 24, 48 hrs, etc.) and running Size Exclusion Chromatography (SEC).

### Results

The dissolution method demonstrated good acceptability to dissolution testing of the peptide PLGA microsphere LAI formulation. Data indicate that 4 g of glass beads yielded the most rapid dissolution of the drug product (FIG. 2), which provided reproducible results over 6 separate runs (FIG. 3). The phosphate buffer at pH 7.4 was biorelevant, but pH 12 was used when accelerated dissolution testing and shorter turnaround time was demanded during development (FIG. 4). However, pH is likely to be dependent on the solute. The specific type and amount of surfactant did not have a significant effect on dissolution (FIGs. 5A-5B), the buffer concentration did not have a profound effect on dissolution (FIG. 6), and media temperature did not appear to have a significant impact on the rate of dissolution (FIG. 8). When 0.02% sodium azide was added into the media, no degradation of released peptide was observed (FIG. 7). The evaporation of media was determined as ~ 1 mL/day/100 mL (data not shown). Based on these results, 33 mM phosphate buffer supplemented with 0.2% TWEEN20 was selected.

### Example 2

The novel method disclosed herein was compared to the conventional USP2 and USP4 methods. When using the USP2 method to dissolve LAI microsphere formulations, the microspheres were observed floating on the surface of the dissolution medium, even though a wetting agent was used to introduce the samples. The microspheres were also easily aggregated and floating around the shaft, which affected sampling and detection. When the USP2 method was applied to hydrogel and liquid crystal formulations, the samples were stacked to the bottom of the vessel wall, which slowed down the drug release due to decreased exposure to the medium.

When USP4 was used to test the same formulations, microbial growth occurred in the vessel, despite addition of sodium azide in the medium. No suitable way has been determined to overcome the growth. The microbial growth led to cleavage of the peptide in the LAI formulation during dissolution and drug release testing, resulting in nonspecific peaks in chromatograms.

With the use of the glass beads to sandwich the LAI samples in microsphere, hydrogel, or liquid crystal, the drug release testing can be smoothly performed under a relatively low stirring speed using a paddle. This also make it possible to use UV fiber optics for in-situ measurement during dissolution without sampling, and enhanced the simplicity and productivity of the testing and decrease variability.

### Example 3

The dissolution methods disclosed herein were used to dissolve a fatty acid-relaxin (FA-RLX) long-acting injection formulation. The suspension of FA-RLX is designed as a long-acting injectable formulation. Particle sizes (PS) of FA-RLX of less than 20 µm, less than 50 µm, and less than 100 µm of FA-RLX were tested. FA-RLX of the indicated particle size was suspended in Miglyol 812N at a concentration of 17.5 mg/mL. 0.5 ml of the respective suspension was then positioned under 0.5 mm glass beads in a 200 mL vessel filled with 50 mL of 4% Hydroxypropyl-β-cyclodextrin in Dulbecco's phosphate buffered saline (PBS). A paddle was positioned above the glass beads, and dissolution occurred at 34 °C, at a stirring rate of 35 rpm. The percent of dissolved FA-RLX was measured by 2^{nd} derivative at 293 - 323 nm (probe path length of 2 mm), every 5 minutes for 24 hours, for a total of 288 readings.

The glass bead under paddle method demonstrated repeatability with suspensions made with PS <20 µm and PS <100 µm FA-RLX formulation (FIG. 9A). Faster release can be observed in the dissolution profiles of the PS <20 µm suspension. In the PS <20 µm suspension, ~90% release was observed after 60 minutes. Slower release can be observed in the dissolution profiles of the PS <100 µm. About 55% release was observed after 60 minutes in the dissolution of the PS <100 µm. Distinction between the dissolution profiles of the PS <20 µm suspension and other dissolution profiles can be observed. However, the PS <100 µm suspension dissolution profile could not be differentiated from the PS <50 µm suspension due to large variability in the dissolution profiles of the PS <50 µm suspension (FIG. 9C).

## Claims

1. An *in vitro* dissolution method comprising agitating a solution placed in a vessel using a mobile paddle; wherein the solution comprises a solute and a media; and wherein the mobile paddle is submerged in the solution;
**characterised in that** the solution further comprises a plurality of beads; wherein the plurality of beads is positioned between the solute and the mobile paddle.

2. The *in vitro* dissolution method of claim 1, comprising, prior to the agitating, loading the solution into the vessel by:
(i) adding the solute to the bottom of the vessel;
(ii) adding the plurality of beads to the bottom of the vessel, wherein the plurality of beads are added on top of the solute; and
(iii) adding a volume of media to the vessel, wherein the media is added on top of the solute and the plurality of beads, optionally wherein the media is added without agitating the solute or the plurality of beads.

3. The *in vitro* dissolution method of any one of the preceding claims, wherein the mobile paddle is not in contact with the plurality of beads or the solute while the mobile paddle is in a stationary position.

4. The *in vitro* dissolution method of any one of the preceding claims, wherein the solute comprises a long acting injectable, optionally wherein the solute comprises a long acting injectable microsphere, optionally wherein the solute comprises a long acting injectable microsphere loaded with a biologically active moiety.

5. The *in vitro* dissolution method of any one of the preceding claims, wherein
(a) the plurality of beads comprises one or more glass beads, one or more plastic beads, one or more silicate beads, one or more metal beads, or any combination thereof; and/or
(b) the plurality of beads comprises one or more beads having a diameter of at least about 0.01 mm, at least about 0.05 mm, at least about 0.1 mm, at least about 0.5 mm, at least about 1.0 mm, at least about 1.5 mm, or at least about 2.0 mm, optionally wherein the plurality of beads comprises one or more beads having a diameter of about 1.0 mm.

6. The *in vitro* dissolution method of any one of the preceding claims, wherein
(a) the volume of the solution in the vessel is sufficient to submerge the mobile paddle, the plurality of beads, and the solute; and/or
(b) the volume of the solution in the vessel is at least about 50 mL, at least about 60 mL, at least about 70 mL, at least about 75 mL, at least about 80 mL, at least about 90 mL, or at least about 100 mL; and/or
(c) the plurality of beads comprises at least about 3 g of beads per about 100 mL of solution, at least about 4 g of beads per 100 mL of about solution, at least about 5 g of beads per about 100 mL of solution, at least about 6 g of beads per about 100 mL of solution, or at least about 7 g of beads per about 100 mL of solution, optionally wherein the plurality of beads comprises about 4 g of beads per 100 mL of solution or less than about 6 g of beads per 100 mL of solution.

7. The *in vitro* dissolution method of any one of the preceding claims, wherein
(a) the solute comprises a long acting injectable microsphere loaded with a biologically active moiety; and/or
(b) the method further comprises applying a cover the vessel during dissolution, optionally wherein the cover reduces or eliminates loss of evaporated solution during dissolution.

8. The *in vitro* dissolution method of any one of the preceding claims, wherein the pH of the solution is adjusted and/or an additional volume of the plurality of beads is added below the solute.

9. The *in vitro* dissolution method of any one of the preceding claims, comprising:
(i) adding a solute to the bottom of a vessel, wherein the solute comprises a long acting injectable microsphere;
(ii) adding a plurality of beads on top of the solute, wherein the plurality of beads comprises one or more glass beads;
(iii) adding a media to the solute and the volume of beads, wherein the media is added without agitating the solute or the volume of beads, and wherein the media is added at a volume that is sufficient to submerge the mobile paddle; and
(iv) applying a force to cause the mobile paddle to spin and/or oscillate, agitating the solution.

10. The *in vitro* dissolution method of any one of the preceding claims, comprising:
(i) adding a first plurality of beads to the bottom of a vessel, wherein the plurality of beads comprises one or more glass beads;
(ii) adding a solute on top of the plurality of beads, wherein the solute comprises a long acting injectable microsphere;
(iii) adding a second plurality of beads on top of the solute, wherein the plurality of beads comprises one or more glass beads;
(iv) adding a media to the solute and the volume of beads, wherein the media is added without agitating the solute or the volume of beads, and wherein the media is added at a volume that is sufficient to submerge the mobile paddle; and
(v) applying a force to cause the mobile paddle to spin and/or oscillate, agitating the solution.

11. The *in vitro* dissolution method of any one of the preceding claims, further comprising adding a volume of a vehicle solution to the solute and/or the plurality of beads, wherein the volume of the vehicle solution is sufficient to wet but not completely submerge the solute and/or the plurality of beads.

12. The *in vitro* dissolution method of any one of the preceding claims, further comprising detecting the presence of dissolved solute in the media;
optionally where the detecting comprises inserting a probe into the solution, obtaining a sample of the solution, or a combination thereof and/or wherein the presence of dissolved solute in the media is detected by probing and/or obtaining a sample from a portion of the solution that is above the mobile paddle.

13. The *in vitro* dissolution method of any one of the preceding claims, wherein the solute comprises a long acting injectable comprising a biologically active moiety;
optionally wherein the biologically active moiety comprises a small molecule or a polypeptide; optionally wherein the polypeptide has a molecular weight of less than about 10 kDa, less than about 9 kDa, less than about 8 kDa, less than about 7 kDa, less than about 6 kDa, less than about 5 kDa, or less than about 4 kDa and/or wherein the polypeptide has a molecular weight of about 4 kDa.

14. The *in vitro* dissolution method of any one of the preceding claims, wherein the solution further comprises;
(a) a surfactant, optionally wherein the surfactant is selected from the group consisting of polysorbate 20, polysorbate 80, TRITON X100, PLURONIC F-68, and any combination thereof; and/or
(b) an antimicrobial agent.

15. A kit comprising:
(i) a vessel;
(ii) a plurality of beads;
(iii) a media;
(iv) a mobile paddle, wherein the mobile paddle can be positioned inside the vessel;
(v) an optional cover; and
(vi) instructions for dissolving a solute according to the method of any one of the preceding claims.

## Patentansprüche

1. In-vitro-Auflösungsverfahren, welches das Rühren einer in einem Gefäß platzierten Lösung unter Verwendung eines beweglichen Paddels umfasst, wobei die Lösung einen gelösten Stoff und ein Medium umfasst und wobei das bewegliche Paddel in der Lösung getaucht ist,
**dadurch gekennzeichnet, dass** die Lösung ferner eine Vielzahl von Perlen umfasst, wobei die Vielzahl von Perlen zwischen dem gelösten Stoff und dem beweglichen Paddel positioniert wird.

2. In-vitro-Auflösungsverfahren nach Anspruch 1, das, vor dem Rühren, das Laden der Lösung in das Gefäß durch Folgendes umfasst:
(i) Hinzugeben der Lösung auf den Boden des Gefäßes,
(ii) Hinzugeben der Vielzahl von Perlen auf den Boden des Gefäßes, wobei die Vielzahl von Perlen oben auf dem gelösten Stoff hinzugegeben wird, und
(iii) Hinzugeben eines Medienvolumens zu dem Gefäß, wobei das Medium oben auf dem gelösten Stoff und der Vielzahl von Perlen hinzugegeben wird, wahlweise, wobei das Medium ohne Rühren des gelösten Stoffs oder der Vielzahl von Perlen hinzugegeben wird.

3. In-vitro-Auflösungsverfahren nach einem der vorhergehenden Ansprüche, wobei sich das bewegliche Paddel nicht in Berührung mit der Vielzahl von Perlen oder dem gelösten Stoff befindet, während sich das bewegliche Paddel in einer unbeweglichen Position befindet.

4. In-vitro-Auflösungsverfahren nach einem der vorhergehenden Ansprüche, wobei der gelöste Stoff einen lang wirkenden injizierbaren Stoff umfasst, wahlweise, wobei der gelöste Stoff eine lang wirkende injizierbare Mikrosphäre umfasst, wahlweise, wobei der gelöste Stoff eine lang wirkende injizierbare Mikrosphäre, die mit einem biologisch aktiven Anteil beladen ist, umfasst.

5. In-vitro-Auflösungsverfahren nach einem der vorhergehenden Ansprüche, wobei
(a) die Vielzahl von Perlen eine oder mehrere Glasperlen, eine oder mehrere Kunststoffperlen, eine oder mehrere Silikatperlen, eine oder mehrere Metallperlen oder eine beliebige Kombination derselben umfasst und/oder
(b) die Vielzahl von Perlen eine oder mehrere Perlen umfasst, die einen Druckmesser von mindestens etwa 0,01 mm, mindestens etwa 0,05 mm, mindestens etwa 0,1 mm, mindestens etwa 0,5 mm, mindestens etwa 1,0 mm, mindestens etwa 1,5 mm oder mindestens etwa 2,0 mm aufweisen, wahlweise, wobei die Vielzahl von Perlen eine oder mehrere Perlen umfasst, die einen Druckmesser von etwa 1,0 mm aufweisen.

6. In-vitro-Auflösungsverfahren nach einem der vorhergehenden Ansprüche, wobei
(a) das Volumen der Lösung in dem Gefäß ausreichend ist, um das bewegliche Paddel, die Vielzahl von Perlen und den gelösten Stoff zu tauchen, und/oder
(b) das Volumen der Lösung in dem Gefäß mindestens etwa 50 ml, mindestens etwa 60 ml, mindestens etwa 70 ml, mindestens etwa 75 ml, mindestens etwa 80 ml, mindestens etwa 90 ml oder mindestens etwa 100 ml beträgt und/oder
(c) die Vielzahl von Perlen mindestens etwa 3 g Perlen je etwa 100 ml Lösung, mindestens etwa 4 g Perlen je etwa 100 ml Lösung, mindestens etwa 5 g Perlen je etwa 100 ml Lösung, mindestens etwa 6 g Perlen je etwa 100 ml Lösung oder mindestens etwa 7 g Perlen je etwa 100 ml Lösung, umfasst, wahlweise, wobei die Vielzahl von Perlen etwa 4 g Perlen je etwa 100 ml Lösung oder weniger als etwa 6 g Perlen je etwa 100 ml Lösung umfasst.

7. In-vitro-Auflösungsverfahren nach einem der vorhergehenden Ansprüche, wobei
(a) der gelöste Stoff eine lang wirkende injizierbare Mikrosphäre, die mit einem biologisch aktiven Anteil beladen ist, umfasst und/oder
(b) das Verfahren ferner das Anwenden einer Abdeckung auf das Gefäß während der Auflösung umfasst, wahlweise, wobei die Abdeckung einen Verlust von verdampfter Lösung während der Auflösung verringert oder ausschließt.

8. In-vitro-Auflösungsverfahren nach einem der vorhergehenden Ansprüche, wobei der pH-Wert der Lösung eingestellt wird und/oder ein zusätzliches Volumen der Vielzahl von Perlen unterhalb des gelösten Stoffs hinzugegeben wird.

9. In-vitro-Auflösungsverfahren nach einem der vorhergehenden Ansprüche, das Folgendes umfasst:
(i) Hinzugeben eines gelösten Stoffs auf den Boden eines Gefäßes, wobei der gelöste Stoff eine lang wirkende injizierbare Mikrosphäre umfasst,
(ii) Hinzugeben einer Vielzahl von Perlen oben auf dem gelösten Stoff, wobei die Vielzahl von Perlen eine oder mehrere Glasperlen umfasst,
(iii) Hinzugeben eines Mediums zu dem gelösten Stoff und dem Volumen von Perlen, wobei das Medium hinzugegeben wird, ohne den gelösten Stoff oder das Volumen von Perlen zu rühren, und wobei das Medium mit einem Volumen hinzugegeben wird, das ausreichend ist, um das bewegliche Paddel zu tauchen, und
(iv) Ausüben einer Kraft, um zu bewirken, dass das bewegliche Paddel sich dreht und/oder schwingt, wobei es die Lösung rührt.

10. In-vitro-Auflösungsverfahren nach einem der vorhergehenden Ansprüche, das Folgendes umfasst:
(i) Hinzugeben einer ersten Vielzahl von Perlen auf den Boden eines Gefäßes, wobei die Vielzahl von Perlen eine oder mehrere Glasperlen umfasst,
(ii) Hinzugeben eines gelösten Stoffs oben auf der Vielzahl von Perlen, wobei der gelöste Stoff eine lang wirkende injizierbare Mikrosphäre umfasst,
(iii) Hinzugeben einer zweiten Vielzahl von Perlen oben auf dem gelösten Stoff, wobei die Vielzahl von Perlen eine oder mehrere Glasperlen umfasst,
(iv) Hinzugeben eines Mediums zu dem gelösten Stoff und dem Volumen von Perlen, wobei das Medium hinzugegeben wird, ohne den gelösten Stoff oder das Volumen von Perlen zu rühren, und wobei das Medium mit einem Volumen hinzugegeben wird, das ausreichend ist, um das bewegliche Paddel zu tauchen, und
(v) Ausüben einer Kraft, um zu bewirken, dass das bewegliche Paddel sich dreht und/oder schwingt, wobei es die Lösung rührt.

11. In-vitro-Auflösungsverfahren nach einem der vorhergehenden Ansprüche, das ferner das Hinzugeben eines Volumens einer Trägerlösung zu dem gelösten Stoff und/oder der Vielzahl von Perlen umfasst, wobei das Volumen der Trägerlösung ausreichend ist, um den gelösten Stoff und/oder die Vielzahl von Perlen zu benetzen, aber nicht vollständig zu tauchen.

12. In-vitro-Auflösungsverfahren nach einem der vorhergehenden Ansprüche, das ferner das Erfassen des Vorhandenseins von aufgelöstem gelöstem Stoff in dem Medium umfasst,
wahlweise, wobei das Erfassen das Einführen einer Sonde in die Lösung, das Gewinnen einer Probe der Lösung oder eine Kombination davon umfasst und/oder wobei das Vorhandensein von aufgelöstem gelöstem Stoff in dem Medium durch Sondieren und/oder Gewinnen einer Probe aus einem Teil der Lösung, der sich oberhalb des beweglichen Paddels befindet, erfasst wird.

13. In-vitro-Auflösungsverfahren nach einem der vorhergehenden Ansprüche, wobei der gelöste Stoff eine lang wirkende injizierbare Mikrosphäre, die einen biologisch aktiven Anteil umfasst, umfasst,
wahlweise, wobei der biologisch aktive Anteil ein kleines Molekül oder ein Polypeptid umfasst, wahlweise, wobei das Polypeptid ein Molekülgewicht von weniger als etwa 10 kDa, weniger als etwa 9 kDa, weniger als etwa 8 kDa, weniger als etwa 7 kDa, weniger als etwa 6 kDa, weniger als etwa 5 kDa oder weniger als etwa 4 kDa aufweist und/oder wobei das Polypeptid ein Molekülgewicht von etwa 4 kDa aufweist.

14. In-vitro-Auflösungsverfahren nach einem der vorhergehenden Ansprüche, wobei die Lösung ferner Folgendes umfasst:
(a) einen grenzflächenaktiven Stoff, wahlweise, wobei der grenzflächenaktive Stoff aus der Gruppe ausgewählt wird, die aus Polysorbat 20, Polysorbat 80, TRITON X100, PLURONIC F-68 und beliebigen Kombinationen davon besteht, und/oder
(b) einen antimikrobiellen Wirkstoff.

15. Kit, das Folgendes umfasst:
(i) ein Gefäß,
(ii) eine Vielzahl von Perlen,
(iii) ein Medium,
(iv) ein bewegliches Paddel, wobei das bewegliche Paddel innerhalb des Gefäßes positioniert werden kann,
(v) eine wahlweise Abdeckung und
(vi) Anweisungen zum Auflösen eines gelösten Stoffs nach dem Verfahren nach einem der vorhergehenden Ansprüche.

## Revendications

1. Procédé de dissolution *in vitro* comprenant l'agitation d'une solution placée dans un récipient en utilisant une pale mobile, dans lequel la solution comprend un soluté et un milieu, et dans lequel la pale mobile est immergée dans la solution ;
**caractérisé en ce que** la solution comprend en outre une pluralité de billes, dans lequel la pluralité de billes est positionnée entre le soluté et la pale mobile.

2. Procédé de dissolution *in vitro* selon la revendication 1, comprenant, avant l'agitation, le chargement de la solution dans le récipient en :
(i) ajoutant le soluté au fond du récipient ;
(ii) ajoutant la pluralité de billes au fond du récipient, dans lequel la pluralité de billes est ajoutée au-dessus du soluté ; et
(iii) ajoutant un volume de milieu dans le récipient, dans lequel le milieu est ajouté au-dessus du soluté et de la pluralité de billes, optionnellement dans lequel le milieu est ajouté sans agiter ni le soluté, ni la pluralité de billes.

3. Procédé de dissolution *in vitro* selon l'une quelconque des revendications précédentes, dans lequel la pale mobile n'est en contact ni avec la pluralité de billes, ni avec le soluté pendant que la pale mobile est dans une position stationnaire.

4. Procédé de dissolution *in vitro* selon l'une quelconque des revendications précédentes, dans lequel le soluté comprend une formulation injectable à action prolongée, optionnellement dans lequel le soluté comprend une microsphère injectable à action prolongée, optionnellement dans lequel le soluté comprend une microsphère injectable à action prolongée chargée avec une fraction biologiquement active.

5. Procédé de dissolution *in vitro* selon l'une quelconque des revendications précédentes, dans lequel
(a) la pluralité de billes comprend une ou plusieurs billes en verre, une ou plusieurs billes en matière plastique, une ou plusieurs billes en silicate, une ou plusieurs billes en métal ou une quelconque combinaison de celles-ci ; et/ou
(b) la pluralité de billes comprend une ou plusieurs billes présentant un diamètre d'au moins environ 0,01 mm, d'au moins environ 0,05 mm, d'au moins environ 0,1 mm, d'au moins environ 0,5 mm, d'au moins environ 1,0 mm, d'au moins environ 1,5 mm ou d'au moins environ 2,0 mm, optionnellement dans lequel la pluralité de billes comprend une ou plusieurs billes présentant un diamètre d'environ 1,0 mm.

6. Procédé de dissolution *in vitro* selon l'une quelconque des revendications précédentes, dans lequel
(a) le volume de la solution dans le récipient est suffisant pour immerger la pale mobile, la pluralité de billes et le soluté ; et/ou
(b) le volume de la solution dans le récipient est d'au moins environ 50 mL, d'au moins environ 60 mL, d'au moins environ 70 mL, d'au moins environ 75 mL, d'au moins environ 80 mL, d'au moins environ 90 mL ou d'au moins environ 100 mL ; et/ou
(c) la pluralité de billes comprend au moins environ 3 g de billes pour environ 100 mL de solution, au moins environ 4 g de billes pour environ 100 mL de solution, au moins environ 5 g de billes pour environ 100 mL de solution, au moins environ 6 g de billes pour environ 100 mL de solution ou au moins environ 7 g de billes pour environ 100 mL de solution, optionnellement dans lequel la pluralité de billes comprend environ 4 g de billes pour 100 mL de solution ou moins d'environ 6 g de billes pour 100 mL de solution.

7. Procédé de dissolution *in vitro* selon l'une quelconque des revendications précédentes, dans lequel
(a) le soluté comprend une microsphère injectable à action prolongée chargée avec une fraction biologiquement active ; et/ou
(b) le procédé comprend en outre l'application d'un moyen de recouvrement sur le récipient pendant la dissolution, optionnellement dans lequel le moyen de recouvrement réduit ou élimine une perte de solution évaporée pendant la dissolution.

8. Procédé de dissolution *in vitro* selon l'une quelconque des revendications précédentes, dans lequel le pH de la solution est réglé et/ou un volume additionnel de la pluralité de billes est ajouté au-dessous du soluté.

9. Procédé de dissolution *in vitro* selon l'une quelconque des revendications précédentes, comprenant :
(i) l'ajout d'un soluté au fond d'un récipient, dans lequel le soluté comprend une microsphère injectable à action prolongée ;
(ii) l'ajout d'une pluralité de billes au-dessus du soluté, dans lequel la pluralité de billes comprend une ou plusieurs billes en verre ;
(iii) l'ajout d'un milieu au soluté et au volume de billes, dans lequel le milieu est ajouté sans agiter ni le soluté, ni le volume de billes, et dans lequel le milieu est ajouté selon un volume qui suffit pour immerger la pale mobile ; et
(iv) l'application d'une force pour faire tourner et/ou osciller la pale mobile, agitant ainsi la solution.

10. Procédé de dissolution *in vitro* selon l'une quelconque des revendications précédentes, comprenant :
(i) l'ajout d'une première pluralité de billes au fond d'un récipient, dans lequel la pluralité de billes comprend une ou plusieurs billes en verre ;
(ii) l'ajout d'un soluté au-dessus de la pluralité de billes, dans lequel le soluté comprend une microsphère injectable à action prolongée ;
(iii) l'ajout d'une seconde pluralité de billes au-dessus du soluté, dans lequel la pluralité de billes comprend une ou plusieurs billes en verre ;
(iv) l'ajout d'un milieu au soluté et au volume de billes, dans lequel le milieu est ajouté sans agiter ni le soluté, ni le volume de billes, et dans lequel le milieu est ajouté selon un volume qui suffit pour immerger la pale mobile ; et
(v) l'application d'une force pour faire tourner et/ou osciller la pale mobile, agitant ainsi la solution.

11. Procédé de dissolution *in vitro* selon l'une quelconque des revendications précédentes, comprenant en outre l'ajout d'un volume d'une solution vectrice au soluté et/ou à la pluralité de billes, dans lequel le volume de la solution vectrice suffit pour mouiller, mais ne pas complètement immerger, le soluté et/ou la pluralité de billes.

12. Procédé de dissolution *in vitro* selon l'une quelconque des revendications précédentes, comprenant en outre la détection de la présence de soluté dissous dans le milieu ;
optionnellement, dans lequel la détection comprend l'insertion d'une sonde dans la solution, l'obtention d'un échantillon de la solution, ou une combinaison de ces actions et/ou dans lequel la présence de soluté dissous dans le milieu est détectée par sondage et/ou obtention d'un échantillon à partir d'une partie de la solution qui est au-dessus de la pale mobile.

13. Procédé de dissolution *in vitro* selon l'une quelconque des revendications précédentes, dans lequel le soluté comprend une formulation injectable à action prolongée comprenant une fraction biologiquement active ;
optionnellement, dans lequel la fraction biologiquement active comprend une petite molécule ou un polypeptide ; optionnellement dans lequel le polypeptide présente un poids moléculaire inférieur à environ 10 kDa, inférieur à environ 9 kDa, inférieur à environ 8 kDa, inférieur à environ 7 kDa, inférieur à environ 6 kDa, inférieur à environ 5 kDa ou inférieur à environ 4 kDa et/ou dans lequel le polypeptide présente un poids moléculaire d'environ 4 kDa.

14. Procédé de dissolution *in vitro* selon l'une quelconque des revendications précédentes, dans lequel la solution comprend en outre :
(a) un surfactant, optionnellement dans lequel le surfactant est sélectionné parmi le groupe constitué du polysorbate 20, du polysorbate 80, du TRITON X100, du PLURONIC F-68 et des combinaisons de ceux-ci ; et/ou
(b) un agent antimicrobien.

15. Kit comprenant :
(i) un récipient ;
(ii) une pluralité de billes ;
(iii) un milieu ;
(iv) une pale mobile, dans lequel la pale mobile peut être positionnée à l'intérieur du récipient ;
(v) un moyen de recouvrement optionnel ; et
(vi) des instructions pour dissoudre un soluté selon le procédé selon l'une quelconque des revendications précédentes.
